# EUROPEAN PATENT APPLICATION

(11) **EP 3 936 523 A1**
(43) Date of publication of application: **12.01.2022**
(21) Application number: 19909467.3
(22) Date of filing: 31.12.2019
(51) Int. Cl.: C07K 16/28, A61K 47/68, A61P 35/00

(54) **DETECTION OF MALIGNANT TUMOR CELLS ANTIBODIES AND USES THEREOF**

(30) Priority: 08.01.2019 CN 201910015053
(71) Applicant: Shenzhen University, Shenzhen, Guangdong 518000 (CN)
(72) Inventor: ZHANG, Suping, Shenzhen, Guangdong 518000 (CN); KIPPS, Thomas James, Shenzhen, Guangdong 518000 (CN); WU, Liufeng, Shenzhen, Guangdong 518000 (CN)
(74) Representative: Snipe, Benjamin Thomas Fletcher
(86) International application number: PCT/CN2019/130486
(87) International publication number: WO 2020/143506

(57) **Abstract**

Disclosed are an anti-ROR2 antibody and a use thereof. The anti-ROR2 antibody includes: a light chain variable region with amino acid sequence of CSASSSVSYMHWYQ, IYDTSKLAS and CQQWSSNPPTFGAG, and a heavy chain variable region with amino acid sequence of YTITSYLMHWV, LEWIGYINPYNDGTKYNEKFKDKAT and CARSDVYYGVRFAYWGQG; a light chain variable region with amino acid sequence of CKASQNVGTNVAWFQ, IYLASYRYS and CQQYNSYPLTFGGG, and a heavy chain variable region with amino acid sequence of YTFTNYWIQWM, LEWIGEINPSNGRTDYNEKFKNRAT and CANYRPGYWGQG; and a light chain variable region with amino acid sequence of CSASSSISYMYWYQ, IYDTSILAS and CQQWSSYPFTFGSG, and a heavy chain variable region with amino acid sequence of YTFTSYLIHWV, LEWIGYINPYNDGTKYNEKFKDKAT and CARSDVYYGVRFAYWGQG.

## Description

### TECHNICAL FIELD

The present disclosure relates to the field of biomedicine, in particular to antibodies for detecting various tumor cells and uses thereof, and in particular to an anti-ROR2 antibody and a use thereof.

### BACKGROUND

The tyrosine kinase orphan-like receptor ROR2 is an important development-related regulatory protein, and ROR2 is highly expressed in various tissues during early embryonic development and plays an important role in tissue growth and differentiation. After the second trimester, the expression level of ROR2 gradually decreases. Except for expression in some osteoblasts and uterine cells, ROR2 is basically not expressed in adult tissues. The current research shows that ROR2 is highly expressed in many tumor tissues, including osteosarcoma, melanoma, colorectal cancer and gastric cancer, breast cancer, lymphoma, leukemia and other cancers. Therefore, many scholars believe that ROR2 is a disease-related gene and can be used as an ideal drug target.

ROR2 is a tyrosine kinase orphan receptor that is expressed in large quantities and plays an important role during embryonic development, and ROR2 is difficult to be detected in most adult tissues. Studies in recent years have found that although most normal tissues do not express ROR2, many malignant tumor cells including breast cancer, lung cancer, pancreatic cancer and other cells have high expression of ROR2. In addition, cancer cells with higher ROR2 expression levels are more malignant and prone to metastasis, recurrence, and poorer prognosis. ROR2 can usually be detected on tumor cells with a low degree of differentiation, which have strong recurrence and metastasis ability.

However, drug development for ROR2 needs to be improved.

### SUMMARY

The present disclosure aims to solve one of the technical problems in the related art to a certain extent. To this end, one purpose of the present disclosure is to provide an anti-ROR2 antibody and a use thereof.

The present disclosure is based on the inventor's following research findings: during the process of tumor research, a surface marker of tumor stem cells, such as CD133, CD44, CD24, ALDH1, or the like, is expressed in tumor cells and normal adult tissues. As a result, the anti-tumor stem cell drugs developed for these surface molecules will also cause certain toxicity to normal tissues. However, ROR2 is also abundantly expressed in tumor stem cells, but less expressed in normal cells. Therefore, the development of therapeutic antibodies against ROR2 will cause less damage to normal tissues. Thus, ROR2 is an ideal drug target. The drugs developed for ROR2 can be used in the treatment of tumors and cancer, and the damage to normal tissues will be relatively small.

To this end, the inventors of the present disclosure developed specific antibodies against ROR2 antigen through research. The antibodies provided in the present disclosure can bind to the ROR2 antigen with high specificity, so that the antibodies can be applied to the targeted therapy of tumors and the specific diagnosis and detection of the ROR2 antigen.

Specifically, the present disclosure provides the following technical solutions:

According to the first aspect of the present disclosure, the present disclosure provides an anti-ROR2 antibody or antigen-binding fragment, including at least one of the following: (1) a light chain variable region with amino acid sequence shown in CSASSSVSYMHWYQ, IYDTSKLAS and CQQWSSNPPTFGAG, and a heavy chain variable region with amino acid sequence shown in YTITSYLMHWV, LEWIGYINPYNDGTKYNEKFKDKAT and CARSDVYYGVRFAYWGQG; (2) a light chain variable region with amino acid sequence shown in CKASQNVGTNVAWFQ, IYLASYRYS and CQQYNSYPLTFGGG, and a heavy chain variable region with amino acid sequence shown in YTFTNYWIQWM, LEWIGEINPSNGRTDYNEKFKNRAT and CANYRPGYWGQG; (3) a light chain variable region with amino acid sequence shown in CSASSSISYMYWYQ, IYDTSILAS and CQQWSSYPFTFGSG, and a heavy chain variable region with amino acid sequence shown in YTFTSYLIHWV, LEWIGYINPYNDGTKYNEKFKDKAT and CARSDVYYGVRFAYWGQG; compared with (1) to (3), the antibody or antigen-binding fragment has an amino acid sequence with at least one conservative amino acid substitution. The antibody or antigen-binding fragment provided herein has high affinity with ROR2 antigen, exhibits affinity at a concentration of 0.0001 µg/ml, and can be used for a variety of ROR2 antigen-related detections. Moreover, it can be applied to the treatment of tumors and play a role in targeted therapy.

According to an embodiment of the present disclosure, the above-mentioned antibody or antigen-binding fragment further includes the following technical features.

In some embodiments of the present disclosure, the antibody or antigen-binding fragment includes at least one of the following: (a) a light chain variable region with amino acid sequence shown in SEQ ID NO: 1 and a heavy chain variable region with amino acid sequence shown in SEQ ID NO: 2; (b) a light chain variable region with amino acid sequence shown in SEQ ID NO: 3 and a heavy chain variable region with amino acid sequence shown in SEQ ID NO: 4; (c) a light chain variable region with amino acid sequence shown in SEQ ID NO: 5 and a heavy chain variable region with amino acid sequence shown in SEQ ID NO: 6; compared with (a) ~ (c), the antibody or antigen-binding fragment has an amino acid sequence with at least one conservative amino acid substitution.

In some embodiments of the present disclosure, the antibody or antigen-binding fragment includes at least one of the following: a light chain with amino acid sequence shown in SEQ ID NO: 7 and a heavy chain with amino acid sequence shown in SEQ ID NO: 8; a light chain with amino acid sequence shown in SEQ ID NO: 9 and a heavy chain with amino acid sequence shown in SEQ ID NO: 10; and a light chain with amino acid sequence shown in SEQ ID NO: 11 and a heavy chain with amino acid sequence shown in SEQ ID NO: 12.

According to the second aspect of the present disclosure, the present disclosure provides an isolated polynucleotide encoding the antibody or antigen-binding fragment described in any one of the embodiments of the first aspect of the present disclosure.

According to an embodiment of the present disclosure, the polynucleotide includes at least one of the following:
a light chain variable region nucleotide sequence shown in SEQ ID NO: 13 and a heavy chain variable region nucleotide sequence shown in SEQ ID NO: 14;
a light chain variable region nucleotide sequence shown in SEQ ID NO: 15 and a heavy chain variable region nucleotide sequence shown in SEQ ID NO: 16;
a light chain variable region nucleotide sequence shown in SEQ ID NO: 17 and a heavy chain variable region nucleotide sequence shown in SEQ ID NO: 18;
a light chain nucleotide sequence shown in SEQ ID NO: 19 and a heavy chain nucleotide sequence shown in SEQ ID NO: 20;
a light chain nucleotide sequence shown in SEQ ID NO: 21 and a heavy chain nucleotide sequence shown in SEQ ID NO: 22;
a light chain nucleotide sequence shown in SEQ ID NO: 23 and a heavy chain nucleotide sequence shown in SEQ ID NO: 24;
compared with any light chain variable region nucleotide sequence described above, the polynucleotide has a sequence of at least 90% homology, optionally a sequence of at least 95% homology, preferably a sequence of at least 98% homology, more preferably a sequence of at least 99% homology;
compared with any heavy chain variable region nucleotide sequence described above, the polynucleotide has a sequence of at least 90% homology, optionally a sequence of at least 95% homology, preferably a sequence of at least 98% homology, more preferably a sequence of at least 99% homology;
compared with any light chain nucleotide sequence described above, the polynucleotide has a sequence of at least 90% homology, optionally a sequence of at least 95% homology, preferably a sequence of at least 98% homology, more preferably a sequence of at least 99% homology;
compared with any heavy chain nucleotide sequence described above, the polynucleotide has a sequence of at least 90% homology, optionally a sequence of at least 95% homology, preferably a sequence of at least 98% homology, more preferably a sequence of at least 99% homology.

According to the third aspect of the present disclosure, the present disclosure provides an expression vector including the polynucleotide described in any one of the embodiments of the second aspect of the present disclosure.

In some embodiments of the present disclosure, the expression vector further includes: a control element operably linked to the polynucleotide for controlling the expression of the polynucleotide in the host cell.

In some embodiments of the present disclosure, the control element includes at least one of the following: a promoter, an enhancer, and a terminator.

In some embodiments of the present disclosure, the host cell is a mammalian cell.

According to the fourth aspect of the present disclosure, the present disclosure provides a recombinant cell including the expression vector described in any one of the embodiments of the third aspect of the present disclosure.

According to the fifth aspect of the present disclosure, the present disclosure provides a method for preparing an anti-ROR2 antibody or antigen-binding fragment, including culturing the recombinant cell described in the fourth aspect of the present disclosure.

According to the sixth aspect of the present disclosure, the present disclosure provides a use of an antibody or antigen-binding fragment in the preparation of a medicine. The medicine is used to treat cancer, and the antibody or antigen-binding fragment is the antibody or antigen-binding fragment described in the first aspect of the present disclosure.

According to the seventh aspect of the present disclosure, the present disclosure provides a use of an antibody or antigen-binding fragment in the preparation of a kit. The kit is used for the diagnosis and detection of ROR2 antigen, and the antibody or antigen-binding fragment is the antibody or antigen-binding fragment described in the first aspect of the present disclosure.

In some embodiments of the present disclosure, the kit is used for immunoblotting, immunoprecipitation, and ELISA detection.

According to the eighth aspect of the present disclosure, the present disclosure provides a pharmaceutical composition, including: the antibody or antigen-binding fragment described in any one of the embodiments of the first aspect of the present disclosure and a pharmaceutically acceptable carrier.

According to the ninth aspect of the present disclosure, the present disclosure provides an anti-ROR2 chimeric antigen receptor, including: an extracellular domain, a transmembrane domain and an intracellular domain. The extracellular domain includes an antibody or antigen-binding fragment, the antibody or antigen-binding fragment is a single chain, and the antibody or antigen-binding fragment is the antibody or antigen-binding fragment according to any one of the embodiments of the first aspect of the present disclosure.

According to the tenth aspect of the present disclosure, the present disclosure provides a Car-T cell that expresses the anti-ROR2 chimeric antigen receptor described in the ninth aspect of the present disclosure.

### BRIEF DESCRIPTION OF THE DRAWINGS

FIG. 1 is an ELISA detection result diagram of Fab antibodies of A12, B22, and B30 at different concentrations according to an embodiment of the present disclosure.
FIG. 2 is an SDS-PAGE identification result diagram of different antibodies according to an embodiment of the present disclosure.
FIG. 3 is an ELISA detection result diagram of chimeric antibodies at different concentrations according to an embodiment of the present disclosure.
FIG. 4 is an SDS-PAGE identification result diagram of ROR2 expression in T47D cells and 231 cells according to an embodiment of the present disclosure.
FIG. 5 is a flow cytometry detection result diagram of different chimeric antibodies for detecting ROR2 in T47D cells according to an embodiment of the present disclosure.
FIG. 6 is a flow cytometry detection result diagram of different chimeric antibodies for detecting ROR2 in 231 cells according to an embodiment of the present disclosure.
FIG. 7 is a flow cytometry detection result diagram of using A12 chimeric antibody to detect the expression of ROR2 in hematological cancer patients according to an embodiment of the present disclosure.
FIG. 8 is a flow cytometry detection result diagram of using A12 chimeric antibody to detect the expression of ROR2 in lymphoma patients according to an embodiment of the present disclosure.
FIG. 9 is an immunoblotting detection result diagram of using different chimeric antibodies according to an embodiment of the present disclosure.
FIG. 10 is an immunoprecipitation detection result diagram of different chimeric antibodies for the detection of endogenous ROR2 protein according to an embodiment of the present disclosure.
FIG. 11 is an immunoprecipitation detection result diagram of different chimeric antibodies for the detection of exogenous ROR2 protein according to an embodiment of the present disclosure.
FIG. 12 is a structural diagram of an A12-car according to an embodiment of the present disclosure.
FIG. 13 is a flow cytometry detection result diagram of a ratio of lymphocytes capable of expressing CAR-T according to an embodiment of the present disclosure.
FIG. 14 is a killing result diagram of A12-Car-T on ROR2 expression-positive T47D cells and ROR2 expression-negative 231 cells according to an embodiment of the present disclosure.

### DETAILED DESCRIPTION OF THE EMBODIMENTS

The embodiments of the present disclosure are described in detail below, and examples of the embodiments are shown in the accompanying drawings. The same or similar reference numerals indicate the same or similar elements or elements with the same or similar functions. The embodiments described below with reference to the drawings are exemplary, and are intended to explain the present disclosure, and should not be understood as a limitation to the present disclosure.

During the process of describing the present disclosure, the relevant terms herein have been explained and described. These explanations and descriptions are only for the convenience of understanding the solution, and should not be regarded as a limitation to the protection solution of the present disclosure.

### Antibody

As used herein, the term "antibody" is an immunoglobulin molecule capable of binding to a specific antigen, including two light chains with a lighter molecular weight and two heavy chains with a heavier molecular weight. The heavy chain (H chain) and the light chain (L chain) are linked by disulfide bonds to form a tetrapeptide chain molecule. The amino acid sequence of the amino terminal (N-terminal) of the peptide chain varies greatly, which is called the variable region (V region), and the carboxyl terminal (C-terminal) is relatively stable with little change, called the constant region (C region). The V regions of the L chain and H chain are called VL and VH, respectively.

In the variable region, the amino acid composition and sequence of certain regions have a higher degree of change, which is called the hypervariable region (HVR). The hypervariable region is where the antigen and the antibody bind, so it is also called the complementarity-determining region (CDR). There are three CDR regions on both the heavy chain variable region and the light chain variable region.

The present disclosure uses the extracellular segment of ROR2 to obtain anti-ROR2 Fab antibody fragments with high specificity and high affinity through immunization. The antibody fragment can specifically bind to the ROR2 antigen, which can target the treatment of tumors and other diseases.

In some embodiments, the present disclosure provides an anti-ROR2 antibody or antigen-binding fragment, including a light chain variable region shown in CSASSSVSYMHWYQ, IYDTSKLAS and CQQWSSNPPTFGAG, and a heavy chain variable region shown in YTITSYLMHWV, LEWIGYINPYNDGTKYNEKFKDKAT and CARSDVYYGVRFAYWGQG. In some other embodiments, the anti-ROR2 antibody or antigen-binding fragment includes a light chain variable region shown in CKASQNVGTNVAWFQ, IYLASYRYS and CQQYNSYPLTFGGG, and a heavy chain variable region shown in YTFTNYWIQWM, LEWIGEINPSNGRTDYNEKFKNRAT and CANYRPGYWGQG. In some another embodiments, the anti-ROR2 antibody or antigen-binding fragment includes a light chain variable region shown in CSASSSISYMYWYQ, IYDTSILAS and CQQWSSYPFTFGSG, and a heavy chain variable region shown in YTFTSYLIHWV, LEWIGYINPYNDGTKYNEKFKDKAT and CARSDVYYGVRFAYWGQG. Take CSASSSVSYMHWYQ as an example. As the CDR region on the variable region of the light chain, it can bind to antigen with a high degree of specificity. The antibody or antigen-binding fragment having the above CDR region can specifically bind to the ROR2 antigen and perform the function of the ROR2 antibody.

In another embodiment, compared with any of the light chain variable regions and heavy chain variable regions described above, the antibody or antigen-binding fragment provided in the present disclosure has at least one conservative amino acid substitution. "Antigen-binding fragment" refers to an antibody fragment that retains the ability to specifically bind to an antigen (ROR2). "Conservative amino acid substitution" refers to the substitution of an amino acid with a residue that is biologically, chemically or structurally similar to another amino acid. Biologically similar means that the substitution does not destroy the biological activity of the ROR2 antibody or the ROR2 antigen. Structurally similar means that amino acids have side chains of similar length, such as alanine, glycine, or serine, or side chains of similar size. Chemically similar means that the amino acids have the same charge or are both hydrophilic or hydrophobic. For example, the hydrophobic residues isoleucine, valine, leucine or methionine are substituted for each other, alternatively, polar amino acids such as arginine for lysine, glutamic acid for aspartic acid, glutamine for asparagine, serine for threonine, and so on.

In some embodiments, the present disclosure provides an antibody or antigen-binding fragment including a light chain variable region sequence shown in SEQ ID NO: 1 and a heavy chain variable region sequence shown in SEQ ID NO: 2. In some other embodiments, compared with the amino acid sequence shown in SEQ ID NO: 1, the light chain variable region sequence of the antibody or antigen-binding fragment has at least one conservative amino acid substitution. In some embodiments, compared with the amino acid sequence shown in SEQ ID NO: 2, the heavy chain variable region sequence of the antibody or antigen-binding fragment has at least one conservative amino acid substitution.

In some embodiments, the present disclosure provides an antibody or antigen-binding fragment including a light chain variable region sequence shown in SEQ ID NO: 3 and a heavy chain variable region sequence shown in SEQ ID NO: 4. In some other embodiments, compared with the amino acid sequence shown in SEQ ID NO: 3, the light chain variable region sequence of the antibody or antigen-binding fragment has at least one conservative amino acid substitution. In some embodiments, compared with the amino acid sequence shown in SEQ ID NO: 4, the heavy chain variable region sequence of the antibody or antigen-binding fragment has at least one conservative amino acid substitution.

In some embodiments, the present disclosure provides an antibody or antigen-binding fragment including a light chain variable region sequence shown in SEQ ID NO: 5 and a heavy chain variable region sequence shown in SEQ ID NO: 6. In some other embodiments, compared with the amino acid sequence shown in SEQ ID NO: 5, the light chain variable region sequence of the antibody or antigen-binding fragment has at least one conservative amino acid substitution. In some embodiments, compared with the amino acid sequence shown in SEQ ID NO: 6, the heavy chain variable region sequence of the antibody or antigen-binding fragment has at least one conservative amino acid substitution.

These conservative amino acid substitutions will not change the biological function of the antibody or antigen-binding fragment. In some specific ways, these conservative amino acid substitutions can occur on amino acids other than the CDR regions in the heavy chain variable region and the light chain variable region.

The antibody including a light chain variable region sequence shown in SEQ ID NO: 1 and a heavy chain variable region sequence shown in SEQ ID NO: 2 is also named as A12 antibody. The light chain variable region sequence VL of the A12 antibody (SEQ ID NO: 1) is: DIVLTQSPAIMSASPGEKVTMTC SASS SVSYMHWYQQKSGTSPKRWIYDTSKLASGVPA RFSGSGSGTSYSLTISSMEAEDAAIYYCQQWSSNPPTFGAGTKLELK. The heavy chain variable region sequence VH of the A12 antibody (SEQ ID NO: 2) is:

QVQLQQSGPELVKPGASVRMSCKAAGYTITSYLMHWVKQRPGQDLEWIGYINPYNDG
TKYNEKFKDKATLTSDKSSSTAYMELSSLTSEDSAVYYCARSDVYYGVRFAYWGQGTL
VTVS.

The antibody including a light chain variable region sequence shown in SEQ ID NO: 3 and a heavy chain variable region sequence shown in SEQ ID NO: 4 is also named as B22 antibody. The light chain variable region sequence VL of the B22 antibody (SEQ ID NO: 3) is: DIVMTQSQKFMSTSVGDRVSVTCKASQNVGTNVAWFQQKPGQSPKPLIYLASYRYSGV PDRFTGSGSGTDFTLTISNVQSEDLAEYFCQQYNSYPLTFGGGTNLEIKR. The heavy chain variable region sequence VH of the B22 antibody (SEQ ID NO: 4) is:

QVQLQQPGAALVKPGASVKLSCKASGYTFTNYWIQWMKQRPGQGLEWIGEINPSNGR
TDYNEKFKNRATLTVDNSSTTAYMQLSSLTSEDSAVYYCANYRPGYWGQGTSVTVSS.

The antibody including a light chain variable region sequence shown in SEQ ID NO: 5 and a heavy chain variable region sequence shown in SEQ ID NO: 6 is also named as B30 antibody. The light chain variable region sequence VL of the B30 antibody (SEQ ID NO: 5) is: DIVLTQSPVIMSASPGEKVTMTCSASSSISYMYWYQQKPGSSPRLLIYDTSILASGVPVR FSGSGSGTSYSLTISRMEAEDAATYYCQQWSSYPFTFGSGTKLEIK. The heavy chain variable region sequence VH of the B30 antibody (SEQ ID NO: 6) is:

QVQLQQSGPELVKPGASVRMSCKAAGYTFTSYLIHWVKQRPGQDLEWIGYINPYNDG
TKYNEKFKDKATLTSDKSSSTAYMELSSLTSEDSAVYYCARSDVYYGVRFAYWGQGTL
VTVSA.

In some embodiments, the present disclosure provides an anti-ROR2 antibody including a light chain shown in SEQ ID NO: 7 and a heavy chain shown in SEQ ID NO: 8.

SEQ ID NO: 7 is:

DIVLTQSPAIMSASPGEKVTMTCSASSSVSYMHWYQQKSGTSPKRWIYDTSK
LASGVPARFSGSGSGTSYSLTISSMEAEDAAIYYCQQWSSNPPTFGAGTKLELKRTVAAP
SVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC.

SEQ ID NO: 8 is:

QVQLQQSGPELVKPGASVRMSCKAAGYTITSYLMHWVKQRPGQDLEWIGY

INPYNDGTKYNEKFKDKATLTSDKSSSTAYMELSSLTSEDSAVYYCARSDVYYGVRFAY
WGQGTLVTVSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK.

In some embodiments, the present disclosure provides an anti-ROR2 antibody including a light chain shown in SEQ ID NO: 9 and a heavy chain shown in SEQ ID NO: 10.

SEQ ID NO: 9 is:

DIVMTQSQKFMSTSVGDRVSVTCKASQNVGTNVAWFQQKPGQSPKPLIYLA
SYRYSGVPDRFTGSGSGTDFTLTISNVQSEDLAEYFCQQYNSYPLTFGGGTNLEIKRTVA
APSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC.

SEQ ID NO: 10 is:

QVQLQQPGAALVKPGASVKLSCKASGYTFTNYWIQWMKQRPGQGLEWIGE
INPSNGRTDYNEKFKNRATLTVDNSSTTAYMQLSSLTSEDSAVYYCANYRPGYWGQGT
SVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK.

In some embodiments, the present disclosure provides an anti-ROR2 antibody including a light chain shown in SEQ ID NO: 11 and a heavy chain shown in SEQ ID NO: 12.

SEQ ID NO: 11 is:

DIVLTQSPVIMSASPGEKVTMTCSASSSISYMYWYQQKPGSSPRLLIYDTSIL
ASGVPVRFSGSGSGTSYSLTISRMEAEDAATYYCQQWSSYPFTFGSGTKLEIKRTVAAPS
VFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC.

SEQ ID NO: 12 is:

QVQLQQSGPELVKPGASVRMSCKAAGYTFTSYLIHWVKQRPGQDLEWIGYI
NPYNDGTKYNEKFKDKATLTSDKSSSTAYMELSSLTSEDSAVYYCARSDVYYGVRFAY
WGQGTLVTVSAASTKGPSVFPLAPSSKSTSGGTAALGCLVKDYFPEPVTVSWNSGALTS

GVHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK.

Polynucleotide, expression vector, recombinant cell

During the process of preparing or obtaining these antibodies, polynucleotides expressing these antibodies can be linked to different vectors and then expressed in different cells to obtain corresponding antibodies.

For this, the present disclosure also provides an isolated polynucleotide encoding the antibody or antigen-binding fragment described above.

In some embodiments, the isolated polynucleotides encode A12 antibody, B22 antibody, and B30 antibody, respectively.

The nucleotide sequence encoding the amino acid sequence shown in SEQ ID NO: 1 of the light chain variable region of the A12 antibody is (SEQ ID NO: 13):

gacatcgtgctgacccagtctccagccatcatgtctgctagccctggcgagaaagtgacaatgacctgctccgcctcctc
ctccgtgtcctacatgcactggtatcagcagaagtccggcacctctcctaagcggtggatctacgacacctccaagctggctagcggagt
gcctgccagattttccggctctggctctggcacctcttactccctgaccatctcctccatggaagccgaggatgccgccatctactactgcc
agcagtggtctagcaaccctcctacctttggcgctggcaccaagctggaactgaag.

The nucleotide sequence encoding the amino acid sequence shown in SEQ ID NO: 2 of the heavy chain variable region of the A12 antibody is (SEQ ID NO: 14):

caggttcagctgcagcagtctggacctgagctggttaagcctggtgcctccgtccggatgtcttgcaaggctgctggcta
caccatcaccagctacctgatgcactgggtcaagcagaggccaggccaggacttggagtggatcggctacatcaacccctacaacgac
ggcaccaagtacaacgagaagttcaaggacaaggctaccctgacctccgacaagtcctcctccaccgcctacatggaactgtccagcct
gacctctgaggactccgccgtgtactactgcgccagatccgatgtgtactatggcgtcagattcgcctactggggccagggcacactggt
cacagtttct.

The nucleotide sequence encoding the amino acid sequence shown in SEQ ID NO: 3 of the light chain variable region of the B22 antibody is (SEQ ID NO: 15):

gacatcgtgctgacccagtctccagccatcatgtctgctagccctggcgagaaagtgacaatgacctgctccgcctcctc
ctccgtgtcctacatgcactggtatcagcagaagtccggcacctctcctaagcggtggatctacgacacctccaagctggctagcggagt
gcctgccagattttccggctctggctctggcacctcttactccctgaccatctcctccatggaagccgaggatgccgccatctactactgcc
agcagtggtctagcaaccctcctacctttggcgctggcaccaagctggaactgaag.

The nucleotide sequence encoding the amino acid sequence shown in SEQ ID NO: 4 of the heavy chain variable region of the B22 antibody is (SEQ ID NO: 16):

caggttcagttgcagcaacctggcgctgctctggttaagcctggcgcttctgtgaagctgtcctgcaaggcttccggctac
accttcaccaactattggatccagtggatgaagcagcggccaggccaaggcctggaatggatcggagagatcaacccttctaacggccg
gaccgactacaacgagaagttcaagaaccgggctaccctgaccgtggacaactcttctaccaccgcctacatgcagctgtccagcctga
cctctgaggactccgccgtgtactactgcgccaactacagacctggctattggggccagggcacctctgtgacagtctcttct.

The nucleotide sequence encoding the amino acid sequence shown in SEQ ID NO: 5 of the light chain variable region of the B30 antibody is (SEQ ID NO: 17):

gacatcgtgatgacccagagccagaaattcatgtccacctccgtgggcgacagagtgtccgtgacatgcaaggcctctc
agaacgtgggcaccaacgtggcctggttccagcagaaacctggccagtctcctaagcctctgatctacctggcctcctaccggtactctg
gcgtgcccgatagattcaccggctctggatctggcaccgacttcaccctgaccatctccaacgtgcagtctgaggacctggccgagtactt
ctgccagcagtacaacagctaccctctgacctttggcggaggcaccaacctggaaatcaag.

The nucleotide sequence encoding the amino acid sequence shown in SEQ ID NO: 6 of the heavy chain variable region of the B30 antibody is (SEQ ID NO: 18):

caggttcagctgcagcagtctggacctgagctggttaagcctggtgcctccgtccggatgtcttgcaaggctgccggcta
caccttcaccagctacctgatccactgggtcaagcagaggccaggccaggacttggagtggatcggctacatcaacccctacaacgacg
gcaccaagtacaacgagaagttcaaggacaaggctaccctgacctccgacaagtcctcctccaccgcctacatggaactgtccagcctg
acctctgaggactccgccgtgtactactgcgccagatccgatgtgtactatggcgtcagattcgcctactggggccagggcacactggtc
acagtttctgct.

The nucleotide sequence encoding the amino acid sequence shown in SEQ ID NO: 7 of the light chain of the A12 antibody is (SEQ ID NO: 19):

[098]	gacatcgtgctgacccagtctccagccatcatgtctgctagccctggcgagaaagtgacaatgacctgctccgcctcctc
ctccgtgtcctacatgcactggtatcagcagaagtccggcacctctcctaagcggtggatctacgacacctccaagctggctagcggagt
gcctgccagattttccggctctggctctggcacctcttactccctgaccatctcctccatggaagccgaggatgccgccatctactactgcc
agcagtggtctagcaaccctcctacctttggcgctggcaccaagctggaactgaagagaacagtggccgctcctagcgtgttcatcttccc
accttccgacgagcagctgaagtctggcacagcctctgtcgtgtgcctgctgaacaacttctaccccagagaagccaaggtgcagtggaa
ggtggacaacgccctgcagagcggcaatagccaagagagcgtgaccgagcaggacagcaaggactctacctacagcctgagcagca
ccctgacactgagcaaggccgactacgagaagcacaaagtgtacgcctgcgaagtgacccaccagggcctttctagccctgtgaccaa
gagcttcaaccggggcgaatgt.

The nucleotide sequence encoding the amino acid sequence shown in SEQ ID NO: 8 of the heavy chain of the A12 antibody is (SEQ ID NO: 20):

caggttcagctgcagcagtctggacctgagctggttaagcctggtgcctccgtccggatgtcttgcaaggctgctggcta
caccatcaccagctacctgatgcactgggtcaagcagaggccaggccaggacttggagtggatcggctacatcaacccctacaacgac
ggcaccaagtacaacgagaagttcaaggacaaggctaccctgacctccgacaagtcctcctccaccgcctacatggaactgtccagcct
gacctctgaggactccgccgtgtactactgcgccagatccgatgtgtactatggcgtcagattcgcctactggggccagggcacactggt
cacagtttctgcctctacaaagggccctagtgtgttccctctggctcccagcagcaagtctacatctggcggaacagccgctctgggctgc

ctggtcaaggattactttcccgagcctgtgaccgtgtcctggaatagcggagcactgacaagcggcgtgcacacctttccagctgtgctgc
aaagcagcggcctgtactctctgagcagcgtggtcacagtgcctagctctagcctgggcacccagacctacatctgcaatgtgaaccaca
agcctagcaacaccaaggtggacaagaaggtggaacccaagagctgcgacaagacccacacctgtcctccatgtcctgctccagaact
gctcggcggaccttccgtgttcctgtttcctccaaagcctaaggacaccctgatgatcagcagaacccctgaagtgacctgcgtggtggtg
gatgtgtcccacgaggatcccgaagtgaagttcaattggtacgtggacggcgtggaagtgcacaacgccaagaccaagcctagagagg
aacagtacaacagcacctacagagtggtgtccgtgctgaccgtgctgcaccaggattggctgaacggcaaagagtacaagtgcaaggt
gtccaacaaggccctgcctgctcctatcgagaaaaccatcagcaaggccaagggccagcctagggaaccccaggtttacacactgcctc
caagcagggacgagctgaccaagaatcaggtgtccctgacctgcctcgtgaagggcttctacccttccgatatcgccgtggaatgggag
agcaatggccagcctgagaacaactacaagacaacccctcctgtgctggacagcgacggctcattcttcctgtacagcaagctgacagtg
gacaagtccagatggcagcagggcaacgtgttcagctgcagcgtgatgcacgaggccctgcacaaccactacacccagaagtccctga
gcctgtctcctggcaaa.

The nucleotide sequence encoding the amino acid sequence shown in SEQ ID NO: 9 of the light chain of the B22 antibody is (SEQ ID NO: 21):

gacatcgtgatgacccagagccagaaattcatgtccacctccgtgggcgacagagtgtccgtgacatgcaaggcctctc
agaacgtgggcaccaacgtggcctggttccagcagaaacctggccagtctcctaagcctctgatctacctggcctcctaccggtactctg
gcgtgcccgatagattcaccggctctggatctggcaccgacttcaccctgaccatctccaacgtgcagtctgaggacctggccgagtactt
ctgccagcagtacaacagctaccctctgacctttggcggaggcaccaacctggaaatcaagagaacagtggccgctcctagcgtgttcat
cttcccaccttccgacgagcagctgaagtctggcacagcctctgtcgtgtgcctgctgaacaacttctaccccagagaagccaaggtgca
gtggaaggtggacaacgccctgcagagcggcaatagccaagagagcgtgaccgagcaggacagcaaggactctacctacagcctga
gcagcaccctgacactgagcaaggccgactacgagaagcacaaagtgtacgcctgcgaagtgacccaccagggcctttctagccctgt
gaccaagagcttcaaccggggcgaatgt.

The nucleotide sequence encoding the amino acid sequence shown in SEQ ID NO: 10 of the heavy chain of the B22 antibody is (SEQ ID NO: 22):

caggttcagttgcagcaacctggcgctgctctggttaagcctggcgcttctgtgaagctgtcctgcaaggcttccggctac
accttcaccaactattggatccagtggatgaagcagcggccaggccaaggcctggaatggatcggagagatcaacccttctaacggccg
gaccgactacaacgagaagttcaagaaccgggctaccctgaccgtggacaactcttctaccaccgcctacatgcagctgtccagcctga
cctctgaggactccgccgtgtactactgcgccaactacagacctggctattggggccagggcacctctgtgacagtctcttctgcctctaca
aagggccctagtgtgttccctctggctcccagcagcaagtctacatctggcggaacagccgctctgggctgcctggtcaaggattactttc
ccgagcctgtgaccgtgtcctggaatagcggagcactgacaagcggcgtgcacacctttccagctgtgctgcaaagcagcggcctgtac
tctctgagcagcgtggtcacagtgcctagctctagcctgggcacccagacctacatctgcaatgtgaaccacaagcctagcaacaccaag
gtggacaagaaggtggaacccaagagctgcgacaagacccacacctgtcctccatgtcctgctccagaactgctcggcggaccttccgt
gttcctgtttcctccaaagcctaaggacaccctgatgatcagcagaacccctgaagtgacctgcgtggtggtggatgtgtcccacgaggat
cccgaagtgaagttcaattggtacgtggacggcgtggaagtgcacaacgccaagaccaagcctagagaggaacagtacaacagcacct
acagagtggtgtccgtgctgaccgtgctgcaccaggattggctgaacggcaaagagtacaagtgcaaggtgtccaacaaggccctgcct

gctcctatcgagaaaaccatcagcaaggccaagggccagcctagggaaccccaggtttacacactgcctccaagcagggacgagctg
accaagaatcaggtgtccctgacctgcctcgtgaagggcttctacccttccgatatcgccgtggaatgggagagcaatggccagcctgag
aacaactacaagacaacccctcctgtgctggacagcgacggctcattcttcctgtacagcaagctgacagtggacaagtccagatggcag
cagggcaacgtgttcagctgcagcgtgatgcacgaggccctgcacaaccactacacccagaagtccctgagcctgtctcctggcaaa.

The nucleotide sequence encoding the amino acid sequence shown in SEQ ID NO: 11 of the light chain of the B30 antibody is (SEQ ID NO: 23):

gacatcgtgctgacccagtctccagtgatcatgtccgcttctcccggcgagaaagtgacaatgacctgctccgcctcctcc
agcatctcctacatgtactggtatcagcagaagcccggctcctctcctcggctgctgatctacgatacctccatcctggcttccggcgtgcc
agtgcggttttctggttctggctctggcacctcctacagcctgaccatctccagaatggaagccgaggacgccgccacctactactgtcag
cagtggtctagctaccccttcacctttggctccggcaccaagctggaaatcaagagaacagtggccgctcctagcgtgttcatcttcccacc
ttccgacgagcagctgaagtctggcacagcctctgtcgtgtgcctgctgaacaacttctaccccagagaagccaaggtgcagtggaaggt
ggacaacgccctgcagagcggcaatagccaagagagcgtgaccgagcaggacagcaaggactctacctacagcctgagcagcaccc
tgacactgagcaaggccgactacgagaagcacaaagtgtacgcctgcgaagtgacccaccagggcctttctagccctgtgaccaagag
cttcaaccggggcgaatgt.

The nucleotide sequence encoding the amino acid sequence shown in SEQ ID NO: 12 of the heavy chain of the B30 antibody is (SEQ ID NO: 24):

caggttcagctgcagcagtctggacctgagctggttaagcctggtgcctccgtccggatgtcttgcaaggctgccggcta
caccttcaccagctacctgatccactgggtcaagcagaggccaggccaggacttggagtggatcggctacatcaacccctacaacgacg
gcaccaagtacaacgagaagttcaaggacaaggctaccctgacctccgacaagtcctcctccaccgcctacatggaactgtccagcctg
acctctgaggactccgccgtgtactactgcgccagatccgatgtgtactatggcgtcagattcgcctactggggccagggcacactggtc
acagtttctgctgcctctacaaagggccctagtgtgttccctctggctcccagcagcaagtctacatctggcggaacagccgctctgggct
gcctggtcaaggattactttcccgagcctgtgaccgtgtcctggaatagcggagcactgacaagcggcgtgcacacctttccagctgtgct
gcaaagcagcggcctgtactctctgagcagcgtggtcacagtgcctagctctagcctgggcacccagacctacatctgcaatgtgaacca
caagcctagcaacaccaaggtggacaagaaggtggaacccaagagctgcgacaagacccacacctgtcctccatgtcctgctccagaa
ctgctcggcggaccttccgtgttcctgtttcctccaaagcctaaggacaccctgatgatcagcagaacccctgaagtgacctgcgtggtgg
tggatgtgtcccacgaggatcccgaagtgaagttcaattggtacgtggacggcgtggaagtgcacaacgccaagaccaagcctagaga
ggaacagtacaacagcacctacagagtggtgtccgtgctgaccgtgctgcaccaggattggctgaacggcaaagagtacaagtgcaag
gtgtccaacaaggccctgcctgctcctatcgagaaaaccatcagcaaggccaagggccagcctagggaaccccaggtttacacactgc
ctccaagcagggacgagctgaccaagaatcaggtgtccctgacctgcctcgtgaagggcttctacccttccgatatcgccgtggaatggg
agagcaatggccagcctgagaacaactacaagacaacccctcctgtgctggacagcgacggctcattcttcctgtacagcaagctgaca
gtggacaagtccagatggcagcagggcaacgtgttcagctgcagcgtgatgcacgaggccctgcacaaccactacacccagaagtccc
tgagcctgtctcctggcaaa.

In some embodiments, the isolated polynucleotide has at least 90% or more homology with the aforementioned amino acid encoding sequence, preferably at least 95% homology, and more preferably 98%, 99% or more homology.

The present disclosure also provides an expression vector, including the isolated polynucleotide described above. When the isolated polynucleotide is linked to the vector, the polynucleotide can be directly or indirectly linked to the control elements on the vector, as long as these control elements can control the translation and expression of the polynucleotide. These control elements can come directly from the vector itself, or can be exogenous, that is, they do not come from the vector itself. These control elements may be promoters, enhancers, terminators, etc., as long as they can regulate gene expression. The polynucleotide only needs to be operably linked to the control elements. "Operably linked" herein refers to linking an exogenous gene to a vector, such that the control elements in the vector, such as transcription control sequences and translation control sequences, etc., can perform their expected functions of regulating the transcription and translation of foreign genes. The polynucleotides encoding the heavy chain and light chain of the antibody can be inserted into different vectors independently, and it is common to insert into the same vector. Commonly used vectors can be, for example, plasmids, bacteriophages, etc., such as pcDNA plasmids.

The present disclosure also provides a recombinant cell including the expression vector. The expression vector can be introduced into mammalian cells (such as 293F cells, CHO cells, etc.) to construct the recombinant cells, and then uses these recombinant cells to express the antibody or antigen-binding fragment provided in the present disclosure. By culturing the recombinant cells, the corresponding antibodies can be obtained.

### Pharmaceutical composition, kit, pharmaceutical use and use in the preparation of kit

The present disclosure also provides a pharmaceutical composition including the antibody or antigen-binding fragment described above and a pharmaceutically acceptable carrier.

The anti-ROR2 antibody provided herein can be incorporated into the pharmaceutical composition suitable for administration to a subject. Generally, the pharmaceutical composition includes the anti-ROR2 antibody provided herein and a pharmaceutically acceptable carrier. The "pharmaceutically acceptable carrier" can include any and all solvents, dispersion media, coatings, antibacterial and antifungal agents, isotonic and absorption delaying agents, and the like that are physiologically compatible. Specific examples may be one or more of water, saline, phosphate buffered saline, glucose, glycerol, ethanol, etc., and combinations thereof. In many cases, isotonic agents are included in the pharmaceutical composition, such as sugars, polyols (such as mannitol, sorbitol), or sodium chloride. The pharmaceutically acceptable carrier can also include minor amounts of auxiliary substances, such as wetting or emulsifying agents, preservatives or buffers, to extend the shelf life or efficacy of the antibody.

For example, the antibody of the present disclosure can be incorporated into the pharmaceutical composition suitable for parenteral administration (e.g., intravenous, subcutaneous, intraperitoneal, intramuscular). The pharmaceutical composition can be prepared into various forms, such as liquid, semi-solid and solid dosage forms, or the like, including but not limited to liquid solution (for example, injection solution and infusion solution), dispersion or suspension, tablet, pill, powder, liposome, and suppository. Typical pharmaceutical composition is in the form of injection solution or infusion solution. The antibody can be administered by intravenous infusion or injection, or intramuscular or subcutaneous injection.

The anti-ROR2 antibody herein can also be made into a part of a kit or other diagnostic reagents as needed. According to an embodiment of the present disclosure, the present disclosure also provides a kit including the ROR2 antibody described above. The kit provided in the present disclosure, for example, can be used for immunoblotting, immunoprecipitation, etc., involving the use of ROR2 antigen and antibody specific binding properties. The kit can include any one or more of the following: antagonist, anti-ROR2 antibody or drug reference material; protein purification column; immunoglobulin affinity purification buffer; cell assay diluent; instruction or literature, or the like. The anti-ROR2 antibody can be used in different types of diagnostic tests, for example, can detect the presence of various diseases or drugs, toxins or other proteins in vitro or in vivo. For example, the subject's serum or blood can be tested to test related diseases. Such related diseases can include ROR2 related diseases, such as various cancers and so on. The antibody provided herein can also be used for radioimmunoassay and radioimmunotherapy of cancer.

These cancers or tumors can be any unregulated cell growth, specifically, lung cancer, gastric cancer, pancreatic cancer, ovarian cancer, liver cancer, breast cancer, colorectal cancer, lymphoma, blood cancer, and so on.

When using the anti-ROR2 antibody provided in the present disclosure to treat cancer, the anti-ROR2 antibody provided in the present disclosure can be provided to the subject. To this end, the present disclosure provides a method for treating cancer, including administering the antibody or antigen-binding fragment thereof provided in the present disclosure to a subject in need.

The solution of the present disclosure will be explained below in conjunction with embodiments. Those skilled in the art will understand that the following embodiments are only used to illustrate the present disclosure, and should not be regarded as limiting the scope of the present disclosure. Where specific techniques or conditions are not indicated in the examples, the procedures shall be carried out in accordance with the techniques or conditions described in the literature in the field or in accordance with the product specification. The reagents or instruments used without the manufacturer's indication are all conventional products that can be purchased commercially.

Through research, the present disclosure has developed ROR2-based antibody-based immunotherapy methods against multiple tumor cells (even tumor stem cells), including ROR2 monoclonal antibody, and a novel ROR2-Car T cell therapy technology.

### Example 1

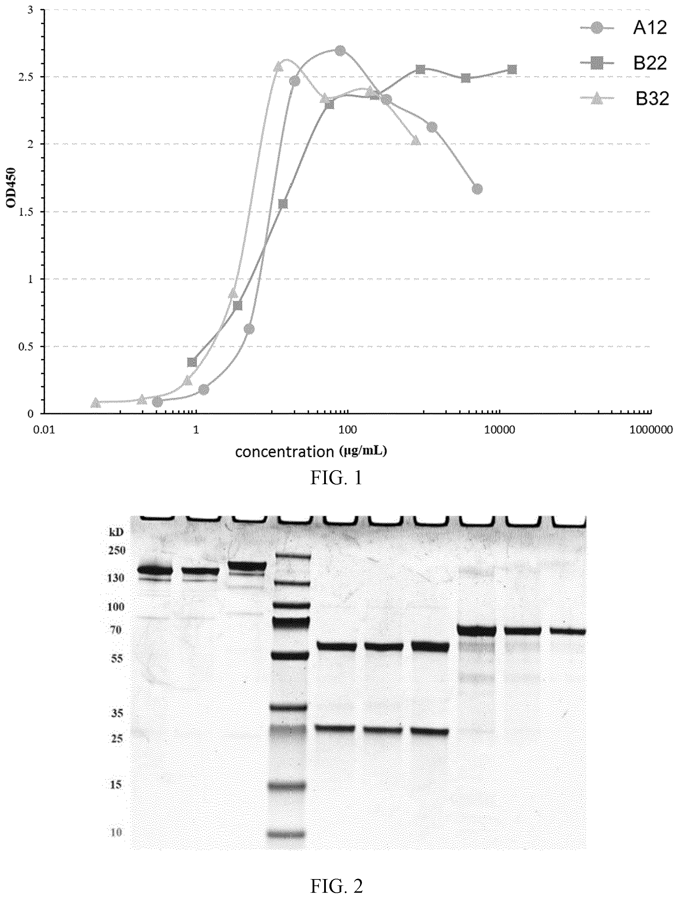

We took ROR2 protein to immunize mice and employed phage library technology to screen and obtain B30, B22 and A12 antibodies. The ELISA detection results of these antibodies are shown in FIG. 1. In FIG. 1, the abscissa represents the concentration of different antibodies, and the ordinate represents the measured OD450 value.

The amino acid sequencing was performed on these antibody samples. The amino acid sequence of the light chain variable region of the antibody and the amino acid sequence of the heavy chain variable region of the antibody were SEQ ID NO: 1 and SEQ ID NO: 2, respectively, and the antibody was named as A12 antibody. The amino acid sequence of the light chain variable region of the antibody and the amino acid sequence of the heavy chain variable region of the antibody were SEQ ID NO: 3 and SEQ ID NO: 4, respectively, and the antibody was named as B22 antibody. The amino acid sequence of the light chain variable region of the antibody and the amino acid sequence of the heavy chain variable region of the antibody were SEQ ID NO: 5 and SEQ ID NO: 6, respectively, and the antibody was named as B30 antibody.

It was detected that the amino acid sequence of the light chain of the A12 antibody was SEQ ID NO: 7, and the amino acid sequence of the heavy chain of the A12 antibody was SEQ ID NO: 8. The amino acid sequence of the light chain of the B22 antibody was SEQ ID NO: 9, and the amino acid sequence of the heavy chain of the B22 antibody was SEQ ID NO: 10. The amino acid sequence of the light chain of the B30 antibody was SEQ ID NO: 11, and the amino acid sequence of the heavy chain of the B30 antibody was SEQ ID NO: 12.

### Example 2

### (1) Expression and purification of ROR2 chimeric antibody

The amino acid sequences of the variable regions of the A12 antibody, the B22 antibody and the B30 antibody were used to deduce their DNA sequences, and the expression sequence of the chimeric antibody was established after species optimization according to the expression vector, and cloned into the pcDNA3.4 vector.

Only codon optimization was performed on the amino acid sequence of the antibody that is not changed after optimization, and the optimized nucleotide sequence encoding the amino acid sequence was easier to be expressed in mammalian cells. Therefore, for example, when the A12 chimeric antibody was expressed, its amino acid sequence was the same as the above-mentioned A12 antibody amino acid sequence, with the difference that the nucleotide sequence encoding the A12 chimeric antibody was optimized.

Specifically, the nucleotide sequence encoding the light chain of the A12 chimeric antibody was SEQ ID NO: 19, and the nucleotide sequence encoding the heavy chain of the A12 chimeric antibody was SEQ ID NO: 20.

The nucleotide sequence encoding the light chain of the B22 chimeric antibody was SEQ ID NO: 21, and the nucleotide sequence encoding the heavy chain of the B22 chimeric antibody was SEQ ID NO: 22.

The nucleotide sequence encoding the light chain of the B30 chimeric antibody was SEQ ID NO: 23, and the nucleotide sequence encoding the heavy chain of the B22 chimeric antibody was SEQ ID NO: 24.

Then the constructed plasmid was transfected into 293F cells with PEI and expressed for 48 hours. After 48 hours, the supernatant was recovered, and purified and chromatographed/concentrated on a Protein A column. The purified protein was identified by SDS-PAGE gel, and the result is shown in FIG. 2. FIG. 2 shows the SDS-PAGE identification results of ROR2-B30 (SA120-1), B22 (SA120-2), and A12 (SA120-3) purified antibodies. The results show that the purified antibodies have high purity.

In FIG. 1, from left to right, the first, second, and third columns represent the SDS-PAGE gel identification results of non-reduced A12, B22, and B30 antibody samples with a loading amount of 2.0 µg. The fourth column represents protein marker. The fifth, sixth, and seventh columns represent the SDS-PAGE gel identification results of reduced A12, B22, and B30 antibody samples with a loading amount of 2.0 µg. The eighth, ninth, and tenth columns represent the SDS-PAGE gel results of reduced fetal bovine serum albumin (BSA) samples with loading amounts of 0.5 µg, 1.0 µg, and 2 µg, respectively.

It can be seen from FIG. 2 that the reduced and non-reduced A12, B22, and B30 chimeric antibody bands displayed in the SDS-PAGE gel are the same. The gray value of the band is close to 2 µg of reduced fetal bovine serum albumin (BSA) sample, which shows that the purified antibody has high purity.

### (2) ELASA verifies the affinity of the purified ROR2 chimeric antibody

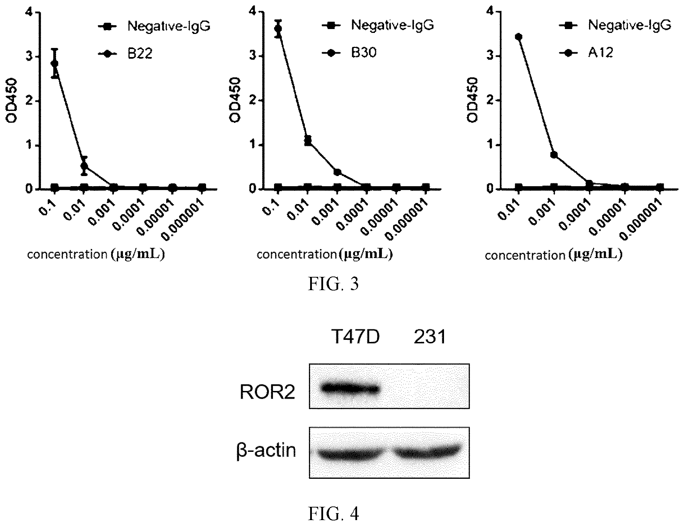

The 96-well plate was coated with ROR2 extracellular protein overnight. After blocking with 5% milk, the purified antibody was diluted to different concentrations and added to different wells for ELASA detection. OD450 result is shown in FIG. 3. FIG. 3 shows the OD450 values of different concentrations of B30 chimeric antibody, B22 chimeric antibody and A12 chimeric antibody. It can be seen that when the concentration of chimeric antibody is low (0.001 µg/ml or 0.0001 µg/ml), the antibody already has affinity, which also shows that the chimeric antibody can be used for ELISA and has a higher affinity.

### (3) Flow cytometry detects the binding capacity of antibody

The purified antibody was subjected to flow cytometry to detect T47D cells (positive ROR2 expression, FIG. 4) and 231 breast cancer cells (negative ROR2 expression, FIG. 4). The results are shown in FIG. 4 to FIG. 6. The flow cytometry results show that the three chimeric antibodies could specifically bind to the ROR2 membrane protein.

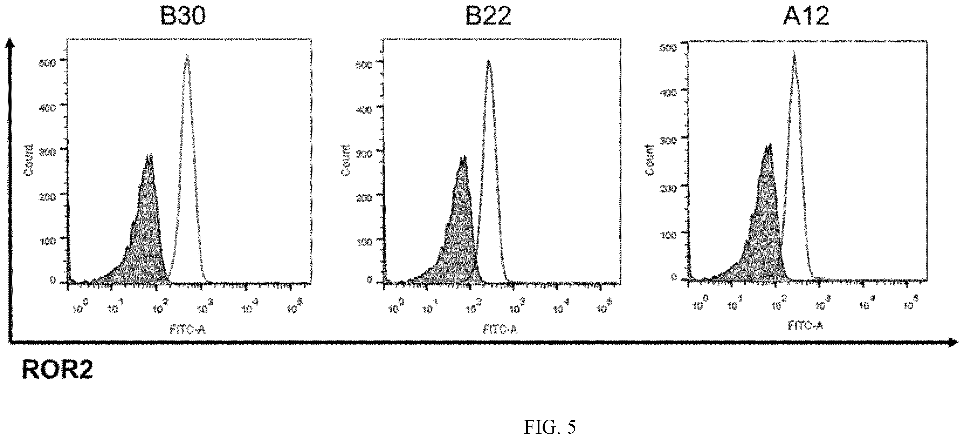

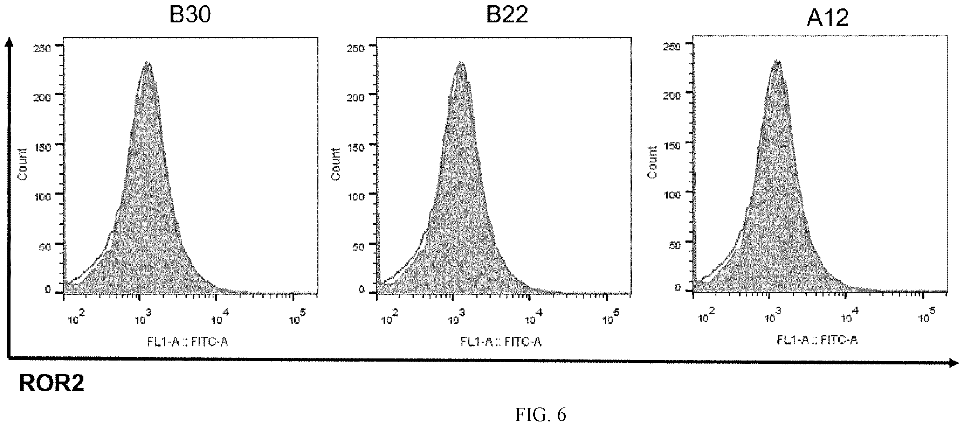

FIG. 4 shows the detection of ROR2 expression levels in T47D cells and 231 cells with ROR2 antibody of the company Santa Cruz. FIG. 5 shows the results of the flow cytometric detection of the ROR2 expression level of T47D with three chimeric antibodies, and the concentrations of the three chimeric antibodies are all 10 µg/ml. FIG. 6 shows the results of the flow cytometric detection of the ROR2 expression level of 231 with three chimeric antibodies, and the concentrations of the three chimeric antibodies are all 10 µg/ml.

### Example 3

### (1) A12 chimeric antibody detecting ROR2 levels of cancer cells in different blood cancer patients and lymphomas

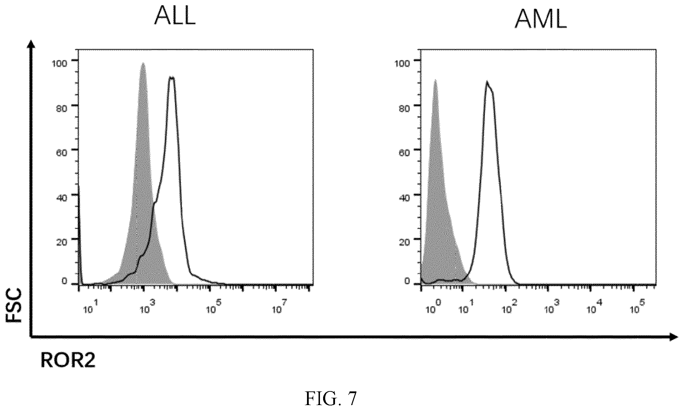

The peripheral blood of patients with different blood cancers were obtained, Ficoll was used to separate the lymphocytes in the blood, and then the lymphocytes were added to the A12 chimeric antibody (concentration of 3 µg/ml), incubated for 1 hour, then Alexa-488-conjugated anti-mouse IgG secondary antibody was incubated and flow cytometry was performed. The results are shown in FIG. 7. In FIG. 7, the results corresponding to ALL represent the flow cytometry results of samples from patients with acute lymphoblastic leukemia, and the results corresponding to AML represent the results of flow cytometry from samples from patients with acute myeloid leukemia.

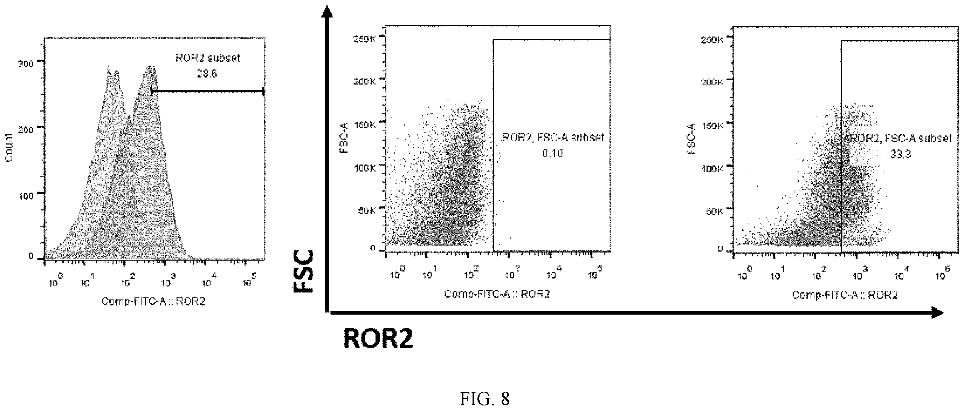

The tissues of lymphoma patients were obtained from the hospital, and then the patiend-derived-xenograft (PDX) model was established. The tumor tissues grown from PDX mice were taken and the tissues were separated into single cells with the human dissociation kit. The single cells were added to the A12 chimeric antibody (concentration of 3 µg/ml), incubated for 1 hour, then Alexa-488-conjugated anti-mouse IgG secondary antibody was incubated, and flow cytometry was performed to check the ROR2 level of tumor cells. The results are shown in FIG 8. The left image of FIG. 8 shows the peak graph of ROR2 fluorescence intensity of tumor cells in lymphoma patients, and the two scatter plots on the right show the distribution of cell fluorescence intensity of tumor cells in blank tubes and B16 antibody stained tumor cells in lymphoma patients.

### (2) ROR2 chimeric antibody can be applied to western blot (WB)

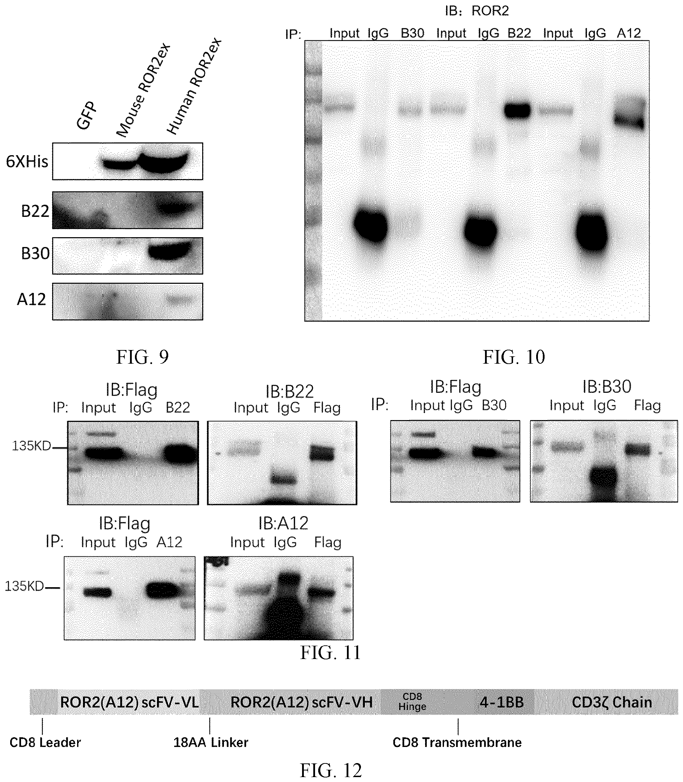

The mouse-derived ROR2 extracellular end (mouse ROR2ex) and human ROR2 extracellular end (human ROR2ex) with His-tagged plasmids and empty GFP were transfected into ROR2 knockout 293T cells (293T-ROR2-KO). After 48 hours, the cells were lysed and the total protein was extracted. After denaturation, the protein was separated by SDS-PAGE gel and subjected to immunoblotting reaction. Three ROR2 chimeric antibodies (5 µg/ml) were used to detect the expression of ROR2. The results are shown in FIG. 9, indicating that the ROR2-B30 chimeric antibody, B22 chimeric antibody and A12 chimeric antibody can all be applied to western blot detection, and can only recognize the extracellular end of the human ROR2 protein.

### (3) ROR2 chimeric antibody can be applied to immunoprecipitation (IP)

In order to determine whether the ROR2 chimeric antibody can be applied to immunoprecipitation (IP), 293T cells with positive ROR2 expression were used for IP. After the cells were lysed, IP was performed with B30 chimeric antibody, B22 chimeric antibody and A12 chimeric antibody, respectively. The protein after IP was detected by immunoblotting with Santa Cruz's ROR2 (Cat. No.: sc-374174) antibody. IgG antibody is used as a negative control antibody (ROR2 cannot be precipitated), and Input is a cell lysate sample that has not been immunoprecipitated (ROR2 protein can be expressed). The results are shown in FIG. 10, indicating that all three antibodies can be applied to IP and can detect endogenous ROR2.

To further verify whether the ROR2 antibody can perform IP, 293T cells were transfected with Flag-tagged pcDNA-ROR2-Flag plasmid. The Flag tag is a polypeptide fragment composed of eight hydrophilic amino acids DYKDDDDK, which can be fused with the protein without occupying the protein epitope or structural domain, avoiding affecting the function, secretion, and transportation of the protein. The Flag tag was used to mark the target protein. If the tag is expressed, the target protein will also be expressed. Then B30 chimeric antibody, B22 chimeric antibody, A12 chimeric antibody and Flag were used for IP, respectively the Flag and B30 chimeric antibody, B22 chimeric antibody, and A12 chimeric antibody were used for immunoblotting (IB). The results are shown in FIG. 11, indicating that regardless of whether it was ROR2 antibody or Flag-labeled antibody for IP, ROR2 (130kD) bands could be detected in the IB results.

It can be seen from the above experiments that by using the spleens of immunized mice to construct a phage library, screening high-affinity monoclonal antibody that specifically recognizes ROR2. Then, the diagnostic function test was performed to verify that the antibody that we screened to specifically recognize ROR2 can be used as a help for the clinically accurate diagnosis of tumor cells, which will lay the foundation for future clinical treatment of ROR2-positive tumor patients, and can also determine the signal pathways affected by the ROR2 antibody, and provide biomarkers for predicting the patient's response to drugs in future clinical trials.

### Example 4

The experiment further verified the ROR2 antibody provided in the present disclosure and developed ROR2-chimeric antigen receptor (CAR) T cell therapy technology for targeted therapy of diseases.

### 1. Construction of ROR2-A12 CAR

The second-generation CAR sequence was used to construct ROR2-CAR with the high-affinity anti-ROR2 antibody A12.

### 2. A12 CAR structure

A12 CAR was composed of CD8 leader, A12 scFv-VL, 18 amino acid linker, A12 scFv-VH, CD8 hinge region, CD8 transmembrane region, 4-1BB costimulatory factor structure, and CD3ζ chain structure, as shown in FIG. 12.

### 3. CAR-T can bind to ROR2

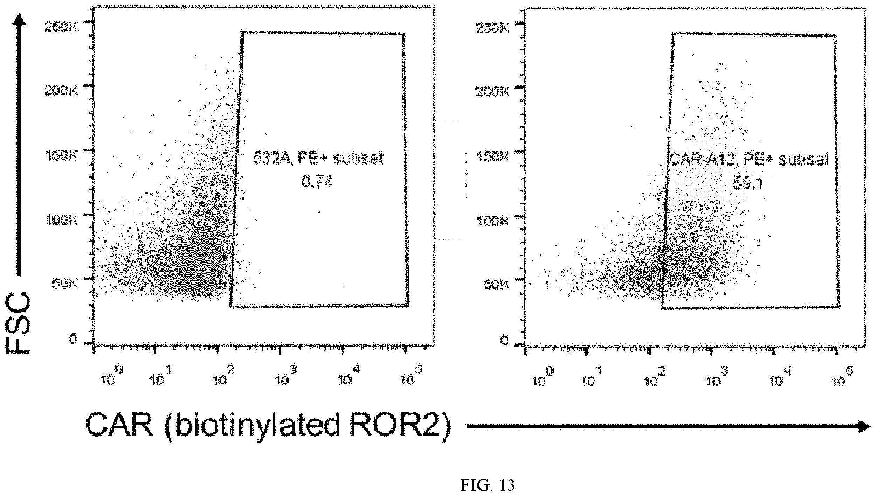

The A12-CAR-T cells were incubated with biotinylated ROR2 protein at a final concentration of 2 µg/ml for 30 minutes on ice. Then, the ROR2 protein was labeled with affinity streptomycin with PE fluorescence and placed on ice for 15 minutes. Flow cytometry was used to detect the ratio of CAR-T that can bind to the ROR2 protein. The flow cytometry results are shown in FIG. 13. In FIG. 13, the left image is a scatter plot of a blank tube, and the right image is a scatter plot of ROR2 protein binding antibody staining, indicating that 59.1% of lymphocytes express A12-CAR T.

### 4. CAR-T kills target cells in vitro

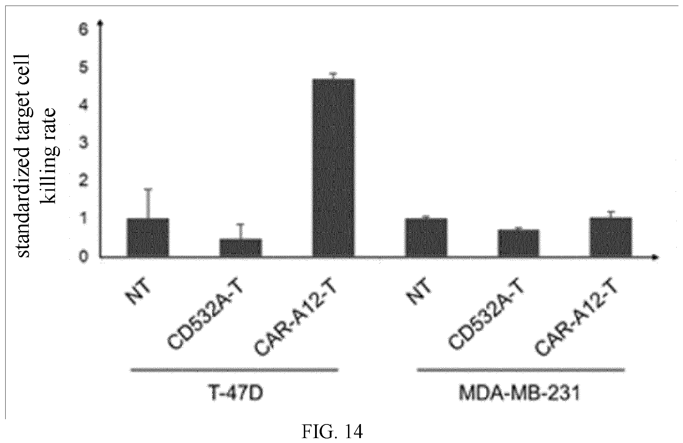

Untransfected T cells (NT), transfected empty CD532Avector T, or transfected A12-CARvector T cells were co-cultured with two different target cells in 96-well plates, respectively. One of the target cells was the T-47D breast cancer cell line that expressed ROR2, and the other of the target cells were the MDA-MB-231 breast cancer cell line that did not express ROR2. 12,000 target cells and 120,000 effect T cells per well, effect (E): target (T)=10: 1, were cultured for 12 hours, and then the lactate dehydrogenase release test was used to detect the killing of the target cells. The killing rate is calculated according to the formula% cytotoxicity = (experimental group-spontaneous effector cell-spontaneous target cell)/(maximum target cell-spontaneous target cell) x 100. The resulting kill rate was then standardized on the basis of the kill rate of the target cells in the untransfected T cell (NT) group. The results are shown in FIG. 14, A12-CAR-T can significantly kill T47D cells with positive ROR2 expression, but cannot kill MDA-MB-231 cells without ROR2 expression.

It can be seen from the above experiments that the development of ROR2-CAR T cell therapy technology uses the high-affinity ROR2 antibodies screened to further develop CAR-T cell therapy technology. There is currently no report on ROR2-CAR T cell therapy at home and abroad.

In the description of the specification, the description of the terms "an embodiment", "some embodiments", "example", "specific example", or "some examples" means that the specific features, structures, materials, or characteristics described in combination with the embodiment or example are included in at least one embodiment or example of the present disclosure. In this specification, the schematic representations of the above-mentioned terms are not necessarily directed to the same embodiment or example. Moreover, the described specific features, structures, materials or characteristics can be combined in any one or more embodiments or examples in a suitable manner. In addition, those skilled in the art can combine the different embodiments or examples and the features of the different embodiments or examples described in this specification without contradicting each other.

Although the embodiments of the present disclosure have been shown and described above, it can be understood that the above-mentioned embodiments are exemplary and should not be construed as limiting the present disclosure. Those of ordinary skill in the art can make changes, modifications, substitutions and modifications to the above-mentioned embodiments within the scope of the present disclosure.

## Claims

1. An anti-ROR2 antibody or antigen-binding fragment, comprising at least one of the following:
(1) a light chain variable region with amino acid sequences of CSASSSVSYMHWYQ, IYDTSKLAS and CQQWSSNPPTFGAG, and a heavy chain variable region with amino acid sequences of YTITSYLMHWV, LEWIGYINPYNDGTKYNEKFKDKAT and CARSDVYYGVRFAYWGQG;
(2) a light chain variable region with amino acid sequences of CKASQNVGTNVAWFQ, IYLASYRYS and CQQYNSYPLTFGGG, and a heavy chain variable region with amino acid sequences of YTFTNYWIQWM, LEWIGEINPSNGRTDYNEKFKNRAT and CANYRPGYWGQG; and
(3) a light chain variable region with amino acid sequences of CSASSSISYMYWYQ, IYDTSILAS and CQQWSSYPFTFGSG, and a heavy chain variable region with amino acid sequences of YTFTSYLIHWV, LEWIGYINPYNDGTKYNEKFKDKAT and CARSDVYYGVRFAYWGQG;
compared with (1) to (3), the antibody or antigen-binding fragment has an amino acid sequence with at least one conservative amino acid substitution;
the anti-ROR2 antibody or antigen-binding fragment comprising at least one of the following:
(a) a light chain variable region with amino acid sequence of SEQ ID NO: 1 and a heavy chain variable region with amino acid sequence of SEQ ID NO: 2;
(b) a light chain variable region with amino acid sequence of SEQ ID NO: 3 and a heavy chain variable region with amino acid sequence of SEQ ID NO: 4; and
(c) a light chain variable region with amino acid sequence of SEQ ID NO: 5 and a heavy chain variable region with amino acid sequence of SEQ ID NO: 6;
compared with (a) to (c), the antibody or antigen-binding fragment has an amino acid sequence with at least one conservative amino acid substitution;
the anti-ROR2 antibody or antigen-binding fragment comprising at least one of the following:
a light chain with amino acid sequence of SEQ ID NO: 7 and a heavy chain with amino acid sequence of SEQ ID NO: 8;
a light chain with amino acid sequence of SEQ ID NO: 9 and a heavy chain with amino acid sequence of SEQ ID NO: 10; and
a light chain with amino acid sequence of SEQ ID NO: 11 and a heavy chain with amino acid sequence of SEQ ID NO: 12.

2. An isolated polynucleotide, wherein the polynucleotide encodes the antibody or antigen-binding fragment of claim 1, and comprises at least one of the following:
a light chain variable region nucleotide sequence of SEQ ID NO: 13 and a heavy chain variable region nucleotide sequence of SEQ ID NO: 14;
a light chain variable region nucleotide sequence of SEQ ID NO: 15 and a heavy chain variable region nucleotide sequence of SEQ ID NO: 16;
a light chain variable region nucleotide sequence of SEQ ID NO: 17 and a heavy chain variable region nucleotide sequence of SEQ ID NO: 18;
a light chain nucleotide sequence of SEQ ID NO: 19 and a heavy chain nucleotide sequence of SEQ ID NO: 20;
a light chain nucleotide sequence of SEQ ID NO: 21 and a heavy chain nucleotide sequence of SEQ ID NO: 22;
a light chain nucleotide sequence of SEQ ID NO: 23 and a heavy chain nucleotide sequence of SEQ ID NO: 24;
compared with any light chain variable region nucleotide sequence described above, the polynucleotide has a sequence of at least 90% homology, a sequence of at least 95% homology, a sequence of at least 98% homology, or a sequence of at least 99% homology;
compared with any heavy chain variable region nucleotide sequence described above, the polynucleotide has a sequence of at least 90% homology, a sequence of at least 95% homology, a sequence of at least 98% homology, or a sequence of at least 99% homology;
compared with any light chain nucleotide sequence described above, the polynucleotide has a sequence of at least 90% homology, a sequence of at least 95% homology, a sequence of at least 98% homology, or a sequence of at least 99% homology;
compared with any heavy chain nucleotide sequence described above, the polynucleotide has a sequence of at least 90% homology, a sequence of at least 95% homology, a sequence of at least 98% homology, or a sequence of at least 99% homology.

3. An expression vector, comprising:
the polynucleotide of claim 2; and
a control element operably connected to the polynucleotide, and for controlling expression of the polynucleotide in a host cell;
wherein the control element comprises at least one of the following: a promoter, an enhancer and a terminator, and the host cell is a mammalian cell.

4. A recombinant cell, comprising the expression vector of claim 3.

5. A method for preparing an anti-ROR2 antibody or antigen-binding fragment, comprising culturing the recombinant cell of claim 4.

6. The use of the antibody or antigen-binding fragment of claim 1 in preparation of a medicine, wherein the medicine is for treatment of cancer.

7. The use of the antibody or antigen-binding fragment of claim 1 in preparation of a kit, wherein the kit is for diagnosis and detection of ROR2 antigen, and the kit is for immunoblotting, immunoprecipitation and/or ELISA detection.

8. A pharmaceutical composition, comprising: the antibody or antigen-binding fragment of claim 1 and a pharmaceutically acceptable carrier.

9. An anti-ROR2 chimeric antigen receptor, comprising: an extracellular domain, a transmembrane domain and an intracellular domain,
wherein the extracellular domain includes the antibody or antigen-binding fragment of claim 1, and the antibody or antigen-binding fragment is a single chain.

10. A CAR-T cell, wherein the CAR-T cell expresses the anti-ROR2 chimeric antigen receptor of claim 9.


SEQUENCE LISTING

<110>  SHENZHEN UNIVERSITY

<120>  ANTIBODIES FOR DETECTING VARIOUS TUMOR CELLS AND USES THEREOF

<130>  20191230

<160>  24

<170>  PatentIn version 3.5

<210>  1
<211>  106
<212>  PRT
<213>  Artificial synthesis

<400>  1

Asp Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Ile Tyr Tyr Cys Gln Gln Trp Ser Ser Asn Pro Pro Thr
                85                  90                  95

Phe Gly Ala Gly Thr Lys Leu Glu Leu Lys
            100                 105

<210>  2
<211>  119
<212>  PRT
<213>  Artificial synthesis

<400>  2

Gln Val Gln Leu Gln Gln Ser Gly Pro Glu Leu Val Lys Pro Gly Ala
1               5                   10                  15

Ser Val Arg Met Ser Cys Lys Ala Ala Gly Tyr Thr Ile Thr Ser Tyr
            20                  25                  30

Leu Met His Trp Val Lys Gln Arg Pro Gly Gln Asp Leu Glu Trp Ile

35                  40                  45

Gly Tyr Ile Asn Pro Tyr Asn Asp Gly Thr Lys Tyr Asn Glu Lys Phe
    50                  55                  60

Lys Asp Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Ser Asp Val Tyr Tyr Gly Val Arg Phe Ala Tyr Trp Gly Gln
            100                 105                 110

Gly Thr Leu Val Thr Val Ser
        115

<210>  3
<211>  108
<212>  PRT
<213>  Artificial synthesis

<400>  3

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Phe Gln Gln Lys Pro Gly Gln Ser Pro Lys Pro Leu Ile
        35                  40                  45

Tyr Leu Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Gly Gly Thr Asn Leu Glu Ile Lys Arg
            100                 105

<210>  4
<211>  114
<212>  PRT
<213>  Artificial synthesis

<400>  4

Gln Val Gln Leu Gln Gln Pro Gly Ala Ala Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Ile Gln Trp Met Lys Gln Arg Pro Gly Gln Gly Leu Glu Trp Ile
        35                  40                  45

Gly Glu Ile Asn Pro Ser Asn Gly Arg Thr Asp Tyr Asn Glu Lys Phe
    50                  55                  60

Lys Asn Arg Ala Thr Leu Thr Val Asp Asn Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Ala Asn Tyr Arg Pro Gly Tyr Trp Gly Gln Gly Thr Ser Val Thr Val
            100                 105                 110

Ser Ser

<210>  5
<211>  106
<212>  PRT
<213>  Artificial synthesis

<400>  5

Asp Ile Val Leu Thr Gln Ser Pro Val Ile Met Ser Ala Ser Pro Gly
1               5                   10                  15

Glu Lys Val Thr Met Thr Cys Ser Ala Ser Ser Ser Ile Ser Tyr Met
            20                  25                  30

Tyr Trp Tyr Gln Gln Lys Pro Gly Ser Ser Pro Arg Leu Leu Ile Tyr
        35                  40                  45

Asp Thr Ser Ile Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Ser Tyr Pro Phe Thr
                85                  90                  95

Phe Gly Ser Gly Thr Lys Leu Glu Ile Lys
            100                 105

<210>  6
<211>  120
<212>  PRT
<213>  Artificial synthesis

<400>  6

Gln Val Gln Leu Gln Gln Ser Gly Pro Glu Leu Val Lys Pro Gly Ala
1               5                   10                  15

Ser Val Arg Met Ser Cys Lys Ala Ala Gly Tyr Thr Phe Thr Ser Tyr
            20                  25                  30

Leu Ile His Trp Val Lys Gln Arg Pro Gly Gln Asp Leu Glu Trp Ile
        35                  40                  45

Gly Tyr Ile Asn Pro Tyr Asn Asp Gly Thr Lys Tyr Asn Glu Lys Phe
    50                  55                  60

Lys Asp Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Ser Asp Val Tyr Tyr Gly Val Arg Phe Ala Tyr Trp Gly Gln
            100                 105                 110

Gly Thr Leu Val Thr Val Ser Ala
        115                 120

<210>  7
<211>  213
<212>  PRT
<213>  Artificial synthesis

<400>  7

Asp Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Ile Tyr Tyr Cys Gln Gln Trp Ser Ser Asn Pro Pro Thr
                85                  90                  95

Phe Gly Ala Gly Thr Lys Leu Glu Leu Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  8
<211>  449
<212>  PRT
<213>  Artificial synthesis

<400>  8

Gln Val Gln Leu Gln Gln Ser Gly Pro Glu Leu Val Lys Pro Gly Ala
1               5                   10                  15

Ser Val Arg Met Ser Cys Lys Ala Ala Gly Tyr Thr Ile Thr Ser Tyr
            20                  25                  30

Leu Met His Trp Val Lys Gln Arg Pro Gly Gln Asp Leu Glu Trp Ile
        35                  40                  45

Gly Tyr Ile Asn Pro Tyr Asn Asp Gly Thr Lys Tyr Asn Glu Lys Phe
    50                  55                  60

Lys Asp Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Ser Asp Val Tyr Tyr Gly Val Arg Phe Ala Tyr Trp Gly Gln
            100                 105                 110

Gly Thr Leu Val Thr Val Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  9
<211>  214
<212>  PRT
<213>  Artificial synthesis

<400>  9

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Phe Gln Gln Lys Pro Gly Gln Ser Pro Lys Pro Leu Ile
        35                  40                  45

Tyr Leu Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Gly Gly Thr Asn Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  10
<211>  444
<212>  PRT
<213>  Artificial synthesis

<400>  10

Gln Val Gln Leu Gln Gln Pro Gly Ala Ala Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Ile Gln Trp Met Lys Gln Arg Pro Gly Gln Gly Leu Glu Trp Ile
        35                  40                  45

Gly Glu Ile Asn Pro Ser Asn Gly Arg Thr Asp Tyr Asn Glu Lys Phe
    50                  55                  60

Lys Asn Arg Ala Thr Leu Thr Val Asp Asn Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Ala Asn Tyr Arg Pro Gly Tyr Trp Gly Gln Gly Thr Ser Val Thr Val
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Lys Lys Val Glu Pro Lys Ser Cys Asp Lys Thr His Thr Cys Pro
    210                 215                 220

Pro Cys Pro Ala Pro Glu Leu Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val

305                 310                 315                 320

Ser Asn Lys Ala Leu Pro Ala Pro Ile Glu Lys Thr Ile Ser Lys Ala
                325                 330                 335

Lys Gly Gln Pro Arg Glu Pro Gln Val Tyr Thr Leu Pro Pro Ser Arg
            340                 345                 350

Asp Glu Leu Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys
        435                 440

<210>  11
<211>  213
<212>  PRT
<213>  Artificial synthesis

<400>  11

Asp Ile Val Leu Thr Gln Ser Pro Val Ile Met Ser Ala Ser Pro Gly
1               5                   10                  15

Glu Lys Val Thr Met Thr Cys Ser Ala Ser Ser Ser Ile Ser Tyr Met
            20                  25                  30

Tyr Trp Tyr Gln Gln Lys Pro Gly Ser Ser Pro Arg Leu Leu Ile Tyr
        35                  40                  45

Asp Thr Ser Ile Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Ser Tyr Pro Phe Thr

85                  90                  95

Phe Gly Ser Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  12
<211>  450
<212>  PRT
<213>  Artificial synthesis

<400>  12

Gln Val Gln Leu Gln Gln Ser Gly Pro Glu Leu Val Lys Pro Gly Ala
1               5                   10                  15

Ser Val Arg Met Ser Cys Lys Ala Ala Gly Tyr Thr Phe Thr Ser Tyr
            20                  25                  30

Leu Ile His Trp Val Lys Gln Arg Pro Gly Gln Asp Leu Glu Trp Ile
        35                  40                  45

Gly Tyr Ile Asn Pro Tyr Asn Asp Gly Thr Lys Tyr Asn Glu Lys Phe
    50                  55                  60

Lys Asp Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys

                85                  90                  95

Ala Arg Ser Asp Val Tyr Tyr Gly Val Arg Phe Ala Tyr Trp Gly Gln
            100                 105                 110

Gly Thr Leu Val Thr Val Ser Ala Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  13
<211>  318
<212>  DNA
<213>  Artificial synthesis

<400>  13
gacatcgtgc tgacccagtc tccagccatc atgtctgcta gccctggcga gaaagtgaca      60

atgacctgct ccgcctcctc ctccgtgtcc tacatgcact ggtatcagca gaagtccggc     120

acctctccta agcggtggat ctacgacacc tccaagctgg ctagcggagt gcctgccaga     180

ttttccggct ctggctctgg cacctcttac tccctgacca tctcctccat ggaagccgag     240

gatgccgcca tctactactg ccagcagtgg tctagcaacc ctcctacctt tggcgctggc     300

accaagctgg aactgaag                                                   318

<210>  14
<211>  357
<212>  DNA
<213>  Artificial synthesis

<400>  14
caggttcagc tgcagcagtc tggacctgag ctggttaagc ctggtgcctc cgtccggatg      60

tcttgcaagg ctgctggcta caccatcacc agctacctga tgcactgggt caagcagagg     120

ccaggccagg acttggagtg gatcggctac atcaacccct acaacgacgg caccaagtac     180

aacgagaagt tcaaggacaa ggctaccctg acctccgaca agtcctcctc caccgcctac 240

atggaactgt ccagcctgac ctctgaggac tccgccgtgt actactgcgc cagatccgat 300

gtgtactatg gcgtcagatt cgcctactgg ggccagggca cactggtcac agtttct 357

<210> 15
<211> 318
<212> DNA
<213> Artificial synthesis

<400> 15
gacatcgtgc tgacccagtc tccagccatc atgtctgcta gccctggcga gaaagtgaca 60

atgacctgct ccgcctcctc ctccgtgtcc tacatgcact ggtatcagca gaagtccggc 120

acctctccta agcggtggat ctacgacacc tccaagctgg ctagcggagt gcctgccaga 180

ttttccggct ctggctctgg cacctcttac tccctgacca tctcctccat ggaagccgag 240

gatgccgcca tctactactg ccagcagtgg tctagcaacc ctcctacctt tggcgctggc 300

accaagctgg aactgaag 318

<210> 16
<211> 342
<212> DNA
<213> Artificial synthesis

<400> 16
caggttcagt tgcagcaacc tggcgctgct ctggttaagc ctggcgcttc tgtgaagctg 60

tcctgcaagg cttccggcta caccttcacc aactattgga tccagtggat gaagcagcgg 120

ccaggccaag gcctggaatg gatcggagag atcaaccctt ctaacggccg gaccgactac 180

aacgagaagt tcaagaaccg ggctaccctg accgtggaca actcttctac caccgcctac 240

atgcagctgt ccagcctgac ctctgaggac tccgccgtgt actactgcgc caactacaga 300

cctggctatt ggggccaggg cacctctgtg acagtctctt ct 342

<210> 17
<211> 321
<212> DNA
<213> Artificial synthesis

<400> 17
gacatcgtga tgacccagag ccagaaattc atgtccacct ccgtgggcga cagagtgtcc 60

gtgacatgca aggcctctca gaacgtgggc accaacgtgg cctggttcca gcagaaacct 120

ggccagtctc ctaagcctct gatctacctg gcctcctacc ggtactctgg cgtgcccgat 180

agattcaccg gctctggatc tggcaccgac ttcaccctga ccatctccaa cgtgcagtct 240

gaggacctgg ccgagtactt ctgccagcag tacaacagct accctctgac ctttggcgga 300

ggcaccaacc tggaaatcaa g 321

<210>  18
<211>  360
<212>  DNA
<213>  Artificial synthesis

<400>  18
caggttcagc tgcagcagtc tggacctgag ctggttaagc ctggtgcctc cgtccggatg      60

tcttgcaagg ctgccggcta caccttcacc agctacctga tccactgggt caagcagagg     120

ccaggccagg acttggagtg gatcggctac atcaacccct acaacgacgg caccaagtac     180

aacgagaagt tcaaggacaa ggctaccctg acctccgaca agtcctcctc caccgcctac     240

atggaactgt ccagcctgac ctctgaggac tccgccgtgt actactgcgc cagatccgat     300

gtgtactatg gcgtcagatt cgcctactgg ggccagggca cactggtcac agtttctgct     360


<210>  19
<211>  639
<212>  DNA
<213>  Artificial synthesis

<400>  19
gacatcgtgc tgacccagtc tccagccatc atgtctgcta gccctggcga gaaagtgaca      60

atgacctgct ccgcctcctc ctccgtgtcc tacatgcact ggtatcagca gaagtccggc     120

acctctccta agcggtggat ctacgacacc tccaagctgg ctagcggagt gcctgccaga     180

ttttccggct ctggctctgg cacctcttac tccctgacca tctcctccat ggaagccgag     240

gatgccgcca tctactactg ccagcagtgg tctagcaacc ctcctacctt tggcgctggc     300

accaagctgg aactgaagag aacagtggcc gctcctagcg tgttcatctt cccaccttcc     360

gacgagcagc tgaagtctgg cacagcctct gtcgtgtgcc tgctgaacaa cttctacccc     420

agagaagcca aggtgcagtg gaaggtggac aacgccctgc agagcggcaa tagccaagag     480

agcgtgaccg agcaggacag caaggactct acctacagcc tgagcagcac cctgacactg     540

agcaaggccg actacgagaa gcacaaagtg tacgcctgcg aagtgaccca ccagggcctt     600

tctagccctg tgaccaagag cttcaaccgg ggcgaatgt                            639


<210>  20
<211>  1347
<212>  DNA
<213>  Artificial synthesis

<400>  20
caggttcagc tgcagcagtc tggacctgag ctggttaagc ctggtgcctc cgtccggatg      60

tcttgcaagg ctgctggcta caccatcacc agctacctga tgcactgggt caagcagagg     120

ccaggccagg acttggagtg gatcggctac atcaacccct acaacgacgg caccaagtac     180

aacgagaagt tcaaggacaa ggctaccctg acctccgaca agtcctcctc caccgcctac     240

atggaactgt ccagcctgac ctctgaggac tccgccgtgt actactgcgc cagatccgat 300

gtgtactatg gcgtcagatt cgcctactgg ggccagggca cactggtcac agtttctgcc 360

tctacaaagg gccctagtgt gttccctctg gctcccagca gcaagtctac atctggcgga 420

acagccgctc tgggctgcct ggtcaaggat tactttcccg agcctgtgac cgtgtcctgg 480

aatagcggag cactgacaag cggcgtgcac acctttccag ctgtgctgca aagcagcggc 540

ctgtactctc tgagcagcgt ggtcacagtg cctagctcta gcctgggcac ccagacctac 600

atctgcaatg tgaaccacaa gcctagcaac accaaggtgg acaagaaggt ggaacccaag 660

agctgcgaca agacccacac ctgtcctcca tgtcctgctc cagaactgct cggcggacct 720

tccgtgttcc tgtttcctcc aaagcctaag gacaccctga tgatcagcag aacccctgaa 780

gtgacctgcg tggtggtgga tgtgtcccac gaggatcccg aagtgaagtt caattggtac 840

gtggacggcg tggaagtgca caacgccaag accaagccta gagaggaaca gtacaacagc 900

acctacagag tggtgtccgt gctgaccgtg ctgcaccagg attggctgaa cggcaaagag 960

tacaagtgca aggtgtccaa caaggccctg cctgctccta tcgagaaaac catcagcaag 1020

gccaagggcc agcctaggga accccaggtt tacacactgc ctccaagcag ggacgagctg 1080

accaagaatc aggtgtccct gacctgcctc gtgaagggct tctacccttc cgatatcgcc 1140

gtggaatggg agagcaatgg ccagcctgag aacaactaca agacaacccc tcctgtgctg 1200

gacagcgacg gctcattctt cctgtacagc aagctgacag tggacaagtc cagatggcag 1260

cagggcaacg tgttcagctg cagcgtgatg cacgaggccc tgcacaacca ctacacccag 1320

aagtccctga gcctgtctcc tggcaaa 1347

<210> 21
<211> 642
<212> DNA
<213> Artificial synthesis

<400> 21
gacatcgtga tgacccagag ccagaaattc atgtccacct ccgtgggcga cagagtgtcc 60

gtgacatgca aggcctctca gaacgtgggc accaacgtgg cctggttcca gcagaaacct 120

ggccagtctc ctaagcctct gatctacctg gcctcctacc ggtactctgg cgtgcccgat 180

agattcaccg gctctggatc tggcaccgac ttcaccctga ccatctccaa cgtgcagtct 240

gaggacctgg ccgagtactt ctgccagcag tacaacagct accctctgac ctttggcgga 300

ggcaccaacc tggaaatcaa gagaacagtg gccgctccta gcgtgttcat cttcccacct 360

tccgacgagc agctgaagtc tggcacagcc tctgtcgtgt gcctgctgaa caacttctac 420

cccagagaag ccaaggtgca gtggaaggtg gacaacgccc tgcagagcgg caatagccaa 480

gagagcgtga ccgagcagga cagcaaggac tctacctaca gcctgagcag caccctgaca 540

ctgagcaagg ccgactacga gaagcacaaa gtgtacgcct gcgaagtgac ccaccagggc 600

ctttctagcc ctgtgaccaa gagcttcaac cggggcgaat gt 642

<210> 22
<211> 1332
<212> DNA
<213> Artificial synthesis

<400> 22
caggttcagt tgcagcaacc tggcgctgct ctggttaagc ctggcgcttc tgtgaagctg 60

tcctgcaagg cttccggcta caccttcacc aactattgga tccagtggat gaagcagcgg 120

ccaggccaag gcctggaatg gatcggagag atcaaccctt ctaacggccg gaccgactac 180

aacgagaagt tcaagaaccg ggctaccctg accgtggaca actcttctac caccgcctac 240

atgcagctgt ccagcctgac ctctgaggac tccgccgtgt actactgcgc caactacaga 300

cctggctatt ggggccaggg cacctctgtg acagtctctt ctgcctctac aaagggccct 360

agtgtgttcc ctctggctcc cagcagcaag tctacatctg gcggaacagc cgctctgggc 420

tgcctggtca aggattactt tcccgagcct gtgaccgtgt cctggaatag cggagcactg 480

acaagcggcg tgcacacctt tccagctgtg ctgcaaagca gcggcctgta ctctctgagc 540

agcgtggtca cagtgcctag ctctagcctg ggcacccaga cctacatctg caatgtgaac 600

cacaagccta gcaacaccaa ggtggacaag aaggtggaac ccaagagctg cgacaagacc 660

cacacctgtc ctccatgtcc tgctccagaa ctgctcggcg gaccttccgt gttcctgttt 720

cctccaaagc ctaaggacac cctgatgatc agcagaaccc ctgaagtgac ctgcgtggtg 780

gtggatgtgt cccacgagga tcccgaagtg aagttcaatt ggtacgtgga cggcgtggaa 840

gtgcacaacg ccaagaccaa gcctagagag gaacagtaca acagcaccta cagagtggtg 900

tccgtgctga ccgtgctgca ccaggattgg ctgaacggca aagagtacaa gtgcaaggtg 960

tccaacaagg ccctgcctgc tcctatcgag aaaaccatca gcaaggccaa gggccagcct 1020

agggaacccc aggtttacac actgcctcca agcagggacg agctgaccaa gaatcaggtg 1080

tccctgacct gcctcgtgaa gggcttctac ccttccgata tcgccgtgga atgggagagc 1140

aatggccagc ctgagaacaa ctacaagaca acccctcctg tgctggacag cgacggctca 1200

ttcttcctgt acagcaagct gacagtggac aagtccagat ggcagcaggg caacgtgttc 1260

agctgcagcg tgatgcacga ggccctgcac aaccactaca cccagaagtc cctgagcctg 1320

tctcctggca aa 1332

<210> 23
<211> 639
<212> DNA
<213> Artificial synthesis

<400>  23
gacatcgtgc tgacccagtc tccagtgatc atgtccgctt ctcccggcga gaaagtgaca      60

atgacctgct ccgcctcctc cagcatctcc tacatgtact ggtatcagca gaagcccggc     120

tcctctcctc ggctgctgat ctacgatacc tccatcctgg cttccggcgt gccagtgcgg     180

ttttctggtt ctggctctgg cacctcctac agcctgacca tctccagaat ggaagccgag     240

gacgccgcca cctactactg tcagcagtgg tctagctacc ccttcacctt tggctccggc     300

accaagctgg aaatcaagag aacagtggcc gctcctagcg tgttcatctt cccaccttcc     360

gacgagcagc tgaagtctgg cacagcctct gtcgtgtgcc tgctgaacaa cttctacccc     420

agagaagcca aggtgcagtg gaaggtggac aacgccctgc agagcggcaa tagccaagag     480

agcgtgaccg agcaggacag caaggactct acctacagcc tgagcagcac cctgacactg     540

agcaaggccg actacgagaa gcacaaagtg tacgcctgcg aagtgaccca ccagggcctt     600

tctagccctg tgaccaagag cttcaaccgg ggcgaatgt                            639


<210>  24
<211>  1350
<212>  DNA
<213>  Artificial synthesis

<400>  24
caggttcagc tgcagcagtc tggacctgag ctggttaagc ctggtgcctc cgtccggatg      60

tcttgcaagg ctgccggcta caccttcacc agctacctga tccactgggt caagcagagg     120

ccaggccagg acttggagtg gatcggctac atcaacccct acaacgacgg caccaagtac     180

aacgagaagt tcaaggacaa ggctaccctg acctccgaca agtcctcctc caccgcctac     240

atggaactgt ccagcctgac ctctgaggac tccgccgtgt actactgcgc cagatccgat     300

gtgtactatg gcgtcagatt cgcctactgg ggccagggca cactggtcac agtttctgct     360

gcctctacaa agggccctag tgtgttccct ctggctccca gcagcaagtc tacatctggc     420

ggaacagccg ctctgggctg cctggtcaag gattactttc ccgagcctgt gaccgtgtcc     480

tggaatagcg gagcactgac aagcggcgtg cacacctttc cagctgtgct gcaaagcagc     540

ggcctgtact ctctgagcag cgtggtcaca gtgcctagct ctagcctggg cacccagacc     600

tacatctgca atgtgaacca caagcctagc aacaccaagg tggacaagaa ggtggaaccc     660

aagagctgcg acaagaccca cacctgtcct ccatgtcctg ctccagaact gctcggcgga     720

ccttccgtgt tcctgtttcc tccaaagcct aaggacaccc tgatgatcag cagaacccct     780

gaagtgacct gcgtggtggt ggatgtgtcc cacgaggatc ccgaagtgaa gttcaattgg     840

tacgtggacg gcgtggaagt gcacaacgcc aagaccaagc ctagagagga acagtacaac     900

agcacctaca gagtggtgtc cgtgctgacc gtgctgcacc aggattggct gaacggcaaa     960

gagtacaagt gcaaggtgtc caacaaggcc ctgcctgctc ctatcgagaa aaccatcagc    1020

aaggccaagg gccagcctag ggaaccccag gtttacacac tgcctccaag cagggacgag 1080

ctgaccaaga atcaggtgtc cctgacctgc ctcgtgaagg gcttctaccc ttccgatatc 1140

gccgtggaat gggagagcaa tggccagcct gagaacaact acaagacaac ccctcctgtg 1200

ctggacagcg acggctcatt cttcctgtac agcaagctga cagtggacaa gtccagatgg 1260

cagcagggca acgtgttcag ctgcagcgtg atgcacgagg ccctgcacaa ccactacacc 1320

cagaagtccc tgagcctgtc tcctggcaaa 1350